# EUROPEAN PATENT APPLICATION

(11) **EP 0 617 130 A2**
(43) Date of publication of application: **28.09.1994**
(21) Application number: 94104620.3
(22) Date of filing: 23.03.1994
(51) Int. Cl.: C12P 41/00

(54) **Method for the resolution of racemic mixtures**

(30) Priority: 24.03.1993 FI 931298
(71) Applicant: ORION-YHTYMÄ OY FERMION, SF-02200 Espoo (FI)
(72) Inventor: Kanerva, Liisa, SF-20660 Littoinen (FI); Sundholm, Oskari, SF-20380 Turku (FI); Kairisalo, Pekka, SF-00950 Helsinki (FI); Hytönen, Martti, SF-02700 Espoo (FI)
(74) Representative: Barz, Peter, Dr.

(57) **Abstract**

The present invention relates to a method for the resolution of racemic mixtures of *threo*-alkyl-2-hydroxy-3-(4-methoxyphenyl)-3-(2-X-phenylthio)-propionates of formula 1 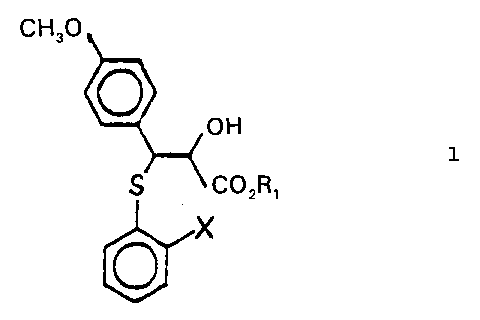
wherein R**₁** is an alkyl group and X is NO**₂**, NH**₂** or an NH-R**₂** group with R**₂** representing an acyl group or an alkoxy carbonyl group, each optionally substituted with one or more halogen atoms, comprising subjecting said racemic mixtures to reaction with an acylation reagent in an essentially anhydrous organic medium in the presence of a lipase.

## Description

The present invention relates to the resolution of racemic mixtures of *threo*-alkyl-2-hydroxy-3-(4-methoxyphenyl)-3-(2-X-phenylthio) propionates.

Optically pure (2S,3S)-threo-alkyl-2-hydroxy-3-(4-methoxyphenyl)-3-(2-X-phenylthio)propionates are valuable intermediates in the synthesis of diltiazem [3; absolute configuration (2S,3S)] which is one of the most promising calcium channel inhibitors in clinical use. 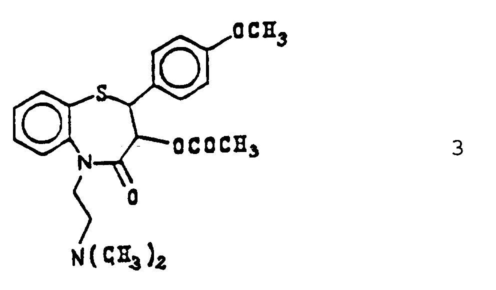
The DE-A 33 37 176 and the US-A 4.908.469 describe the separation of (±)-2-hydroxy-3-(4-methoxyphenyl)-3-(2-aminophenylthio)propionic acid and propionates. Senuma et al. (Chem. Pharm. Bull. 37, 3204-3208; 1989) describe the separation of the corresponding enantiomers of (2-nitrophenylthio)propionic acid based on the formation of the diastereomeric salts with optically active aminoacids (L-lysine) or tartaric acid. The chemical resolution similar to the formation of diastereomers requires the availability of an optically pure compound, such as L-lysine, dismounting of the diastereomer and isolation of the liberated L-lysine, for example, and recycling it from one solution to another.

So far the only biocatalytical method for the preparation of (2S,3S)-threo-alkyl-2-hydroxy-3-(4-methoxyphenyl)-3-(2-X-phenylthio)propionates describes the hydrolysis of racemic threo-methyl-2-acyloxy-3-(4-methoxyphenyl)-3-(2-nitrophenyl-thio)propionate catalyzed by lipase (Chem. Pharm. Bull. 37, 2876-2878; 1989). The enzymatic hydrolysis proceeds slowly because the starting material and the products dissolve poorly in water. This is why the reactions have to be performed in an organic solvent which is saturated with water. Additionally, the reaction is kinetically controlled. Thus, the yield of the optically active product remains low, if its highest possible optical purity is to be reached.

Accordingly, it is the object of the present invention to provide a simple and highly enantiospecific enzymatic method for the resolution of racemic *threo*-alkyl-2-hydroxy-3-(4-methoxyphenyl)-3-(2-X-phenylthio)propionates in order to obtain the optical isomers with high purity.

The present invention therefore concerns a method for the resolution of racemic mixtures of *threo*-alkyl-2-hydroxy-3-(4-methoxyphenyl)-3-(2-X-phenylthio)propionates of formula 1 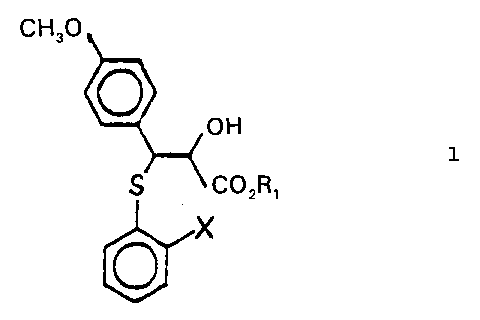
wherein R**₁** is an alkyl group and X is NO**₂**, NH**₂** or an NH-R**₂** group with R**₂** representing an acyl group or an alkoxy carbonyl group, each optionally substituted with one or more halogen atoms, comprising subjecting said racemic mixtures to reaction with an acylation reagent in an essentially anhydrous organic medium in the presence of a lipase.

As used herein, formula 1 is meant to represent both racemic mixtures and the individual optical isomers.

In the definitions as used herein, alkyl is preferably a C**₁₋₆**-alkyl group, such as methyl, ethyl, propyl, isopropyl, butyl, pentyl or hexyl;
acyl is preferably a C**₁₋₄**-acyl group, optionally substituted with one or more, in particular 1 to 3 halogen atoms, such as formyl, acetyl, trifluoroacetyl, propionyl or butyryl;
alkoxy is preferably a C**₁₋₄**-alkoxy group, optionally substituted with one or more, in particular 1 to 3 halogen atoms, such as methoxy, ethoxy, propoxy or butoxy;
halogen may be F, Cl, Br and I.

Most preferably, X is NO**₂**, NH**₂**, NHCHO, NHCOCH**₃**, NHCOCF**₃** or NHCO**₂**CH**₃**.

In the method of the present invention the (2R,3R)-enantiomer of the racemic mixture of compound 1 is allowed to react with a suitable acylation reagent in the presence of a lipase to result in the acylated (2R,3R)-threo-alkyl-2-acyloxy-3-(4-methoxyphenyl)-3-(2-X-phenylthio)-propionates according to formula 2 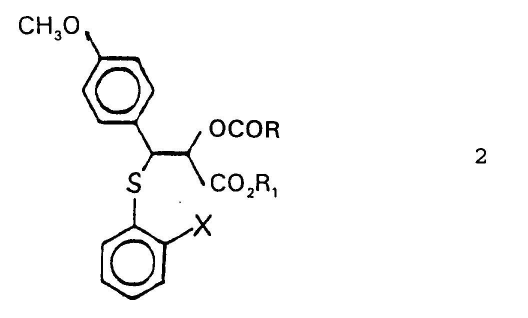
wherein R is an alkyl group and X and R**₁** have the meaning as defined above.

The acylated compounds of formula 2 can be separated from the unreacted (2S,3S)-1 enantiomer, suitably chromatographically or using fractional crystallization from diethyl ether or from toluene. If desired, the acylated (2R,3R)-enantiomers of formula 2 can be deacylated according to known methods.

The acylation reagent according to the invention may be any acylation reagent which is a suitable substrate for the lipase and effective enough to acylate a sterically hindered alcohol of formula 1 so that the reaction is completed within a reasonable time. It is also desirable that the acylation reagent favours an equilibrium of the transesterification reaction which is on the product side as completely as possible.

Suitable acylation reagents are acid anhydrides such as acetic acid anhydride, propionic anhydride and butyric anhydride, oxime esters such as acetoneoxim acetate, vinyl esters such as vinyl acetat and vinyl propionate, carboxylic acid esters such as ethyl acetate, and/or activated esters such as 2,2,2-trifluoro-ethylacetate.

Lipases are used as a biocatalyst in the transesterification reaction. Suitable lipases are, for instance, commercially available lipases such as from *Mucor miehei* (Biocatalysts), *Candida lipolytica* (Biocatalysts), *Candida cylindracea* (Sigma), *Candida cylindracea* (AY 30 Amano), *Aspergillus niger* (AP-6, Amano) and *Pseudomonas cepacia* (lipase PS, Amano). Preferably a lipase of *Pseudomonas cepacia* is used.

The lipases can be used either as such, for example as a powder, or immobilized on a solid support. Preferably the lipase is used on a solid support as the activity of an immobilized lipase such as lipase PS is multiple when compared to the activity of an untreated enzyme and the activity of the immobilized enzyme lasts longer in reuse.

Suitable supports are based, for example, on diatomaceous earth or kieselguhr. Such adsorbents are commercially available, for instance under the trade names Celite® and Chromosorb®.

The reaction is carried out in an essentially anhydrous organic medium which is suitably chosen so as to be capable to dissolve both the starting compounds of formula 1 and the compounds of formula 2 resulting from the acylation reaction. The enzymes themselves do not dissolve in the organic medium. The term "organic medium" as used herein is meant to comprise individual solvents as well as mixtures of different solvents.

A large variety of organic solvents can be used. Suitable solvents, for example, are tetrahydrofurane (THF), diethyl ether, diisopropyl ether and toluene. Some of the acylation reagents such as vinyl esters and ethyl acetate, may also advantageously serve as the solvent. Preferred solvents are diethyl ether and toluene, because the unreacted (2S,3S)-enantiomer of formula (1) can thus be isolated after concentration by fractional crystallization, when the enzyme has first been removed by filtration from the reaction mixture.

The reaction is carried out under mild conditions, suitably in a temperature range of from 20°C to 60°C.

In case where X in position 2 of the thiophenol ring is an NH**₂** group, there always occurs some acylation of the NH**₂** group unenzymatically together with the enzymatic acylation. For that reason, from the point of view of the quality of the final product, better results are obtained, if the NH**₂** group is protected before the resolution with a suitable protective group, for instance a formyl group, an acetyl group, a trifluoroacetyl group or a methoxy carbonyl group. Protection of the amino group and removal of the protection group after the resolution are performed according to known methods.

The valuable drug substance diltiazem 3 is obtained from the (2S,3S)-enantiomer of formula 1 by lactamizing the (2S,3S)-acid according to formula 4 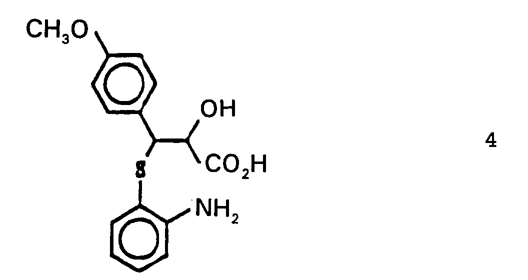
after removing the protective groups. In the case where the residue X in position 2 in the thiophenol ring of compound 1 is an NO**₂** group, the NO**₂** group has to be reduced to an NH**₂** group for the synthesis of diltiazem.

The resulting lactame of formula 5a 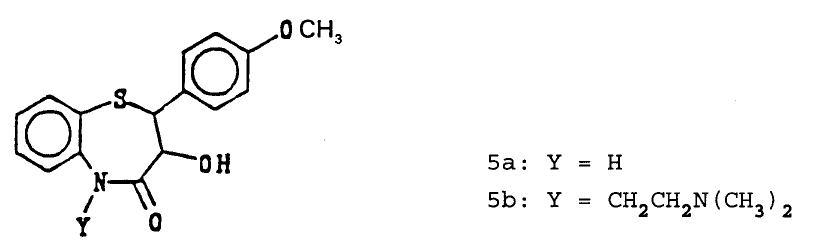
is aminoalkylated by using, for example, a mixture of toluene and N-methyl-pyrolidin-2-one as solvent and potassium carbonate as base. The intermediate 5b obtained by this aminoalkylation reaction is suitably acylated with acetic anhydride and the obtained diltiazem 3 can be crystallized as its hydrochloride from ethanol.

One advantage of the method of the present invention is the stability of the compounds of formula 1 with regard to the subsequent reactions. By using the method of the present invention stereochemically pure (2S,3S)-enantiomers of formula 1 can be prepared by applying the specifity of lipase when acylating the corresponding (2R,3R)-enantiomers in organic solvents. The lipases act practically enantiospecifically, i. e. the reaction stops at 50% conversion and the products of the resolution, the unreacted enantiomer of the racemic mixture, the (2S,3S)-enantiomer of formula 1, and the acylated (2R,3R)-enantiomer of formula 2 can be isolated as optically pure substances with almost 100% yield. The yields of the reactions are excellent under mild conditions. Furthermore, as the biocatalytic resolution of the present invention is based on the selective reaction of the "wrong" enantiomer of the racemate with the achiral substrate, the drawbacks associated with the formation of diastereomers are avoided.

The present invention is further illustrated by the following examples.

### Example 1

### Specificity of lipase PS and acylation of racemic alcohols

### Method A

1 ml of the organic solvent, which is 0.05 M with respect to one of the racemic alcohols according to formula 1, wherein X = NO**₂** (compound **1a**), NH**₂** (**1b**), NHCOCH**₃** (**1c**), NHCOCF**₃** (**1d**), NHCO**₂**CH**₃** (**1e**) and NHCHO (**1f**) and wherein R**₁** = CH**₃** or CH**₂**CH**₃**, and 0.2 M with respect to the acylating reagent, i.e. vinyl or acetoneoxime acetate or acetic acid anhydride or butyric anhydride, is added to the reaction vessel, which contains a certain amount of commercially available lipase PS. The assay of the enzyme is presented in table 1. The reaction mixture is shaken effectively in a shaker at a temperature as indicated in table 1. The proceeding of the reaction is followed by sampling the reaction mixture at suitable intervals, the enzyme is removed by filtering and then the sample is injected for liquid chromatography (column: C-18, eluent: MeOH/H**₂**O 70/30).

The reaction stops at approx. 50% conversion. The excess of the unreacted (2S,3S)-enantiomer (enantiomeric excess, e.e) of formula 1 is determined by HPLC (column: Chiralcel OG; eluent: hexane/isopropylalcohol 70/30) directly from the sample taken from the reaction mixture; in the case of compounds **1a**, **b and c** the enantiomers do not separate perfectly under the HPLC conditions. This is why the e.e. values have been determined also in the presence of a tris [3-heptafluoropropylhydroxymethylene)-(+)-camphorato] europium III [Eu(hfc)**₃**] derivative using **¹**H NMR spectroscopy.

### Method B

The used enzyme is immobilized on a solid support before using. For that reason 0.4 g of lipase PS is dissolved on icebath in 15 ml of 20 mM of Tris-HCl buffer (pH 8.0). 240 mg of saccharose and 4 g of the support substance [Celite® (Aldrich) or Chromosorb® 101 (80-100 mesh, Sigma)] are added thereto. The mixture is agitated for 10 - 15 minutes. The enzyme preparation is allowed to dry on glass at room temperature.

The acylation of compounds **1a-f** (R**₁** = CH**₃**) with vinyl acetate is performed as described in method A except that such an amount of the enzyme preparation is weighed, that the amount of the lipase PS contained corresponds to the amounts given in table 1.

Results from methods A and B are presented in Table 1.

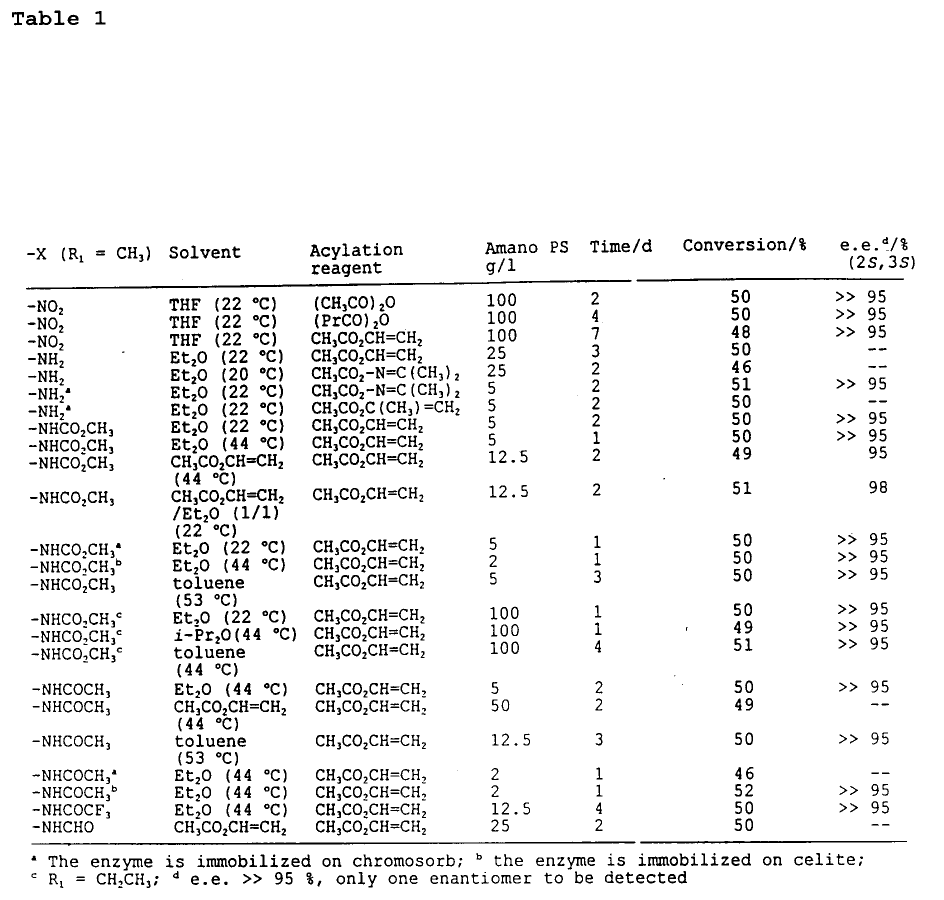

### Example 2

### Preparation of (2S,3S)-1a and (2R,3R)-2a

1.1 g (0.0030 mol) of racemic compound **1a** (X = NO**₂**, R**₁** = CH**₃**) and 0.80 ml (0.0085 mol) of acetic anhydride dissolved in 60 ml of THF is added to the reaction vessel, which contains 6 g of enzyme (lipase PS). The reaction mixture is shaken at room temperature for two days, after which the reaction has stopped. The enzyme is separated by filtering and THF is evaporated off. The residue which is dissolved in dichloromethane is washed with saturated NaHCO**₃** solution and water. (2S,3S)-**1a** and (2R,3R)-**2a** (X = NO**₂**, R = CH**₃**) are separated flash-chromatographically using a mixture of CH**₂**Cl**₂**/hexane/AcOEt (63/31/6) as an eluent. As a result, 0.56 g (0.0015 mol) of unreacted starting material (2S,3S)-**1a**, which is an optically pure product as determined by the HPLC method of example 1, and 0.63 g (0.0015 mol) of (2R,3R)-**2a** enantiomer (R = CH**₃**) are obtained. Both compounds are optically pure (e.e. >> 95 %) also by **¹**H NMR spectroscopic determination, (**1a**, Eu(hfc)**₃**:CO**₂**Me, d, **δ** = 3.84 and 3.94 used as a shifting reagent).

In order to determine the absolute configuration of the above prepared optically pure compound **1a**, the enantiomer is hydrolyzed to the corresponding acid. 0.39 g of the above separated enantiomer **1a** is dissolved in 15 ml of a mixture of methanol and NaOH solution (0.5 M) 2/1. When the hydrolysis is completed the acid formed is precipitated by rendering the reaction mixture acidic with hydrochloric acid. The melting point of the resulting acid is 113 - 114°C and 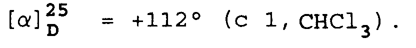
The corresponding literature values for the (2S,3S) - enantiomer of the acid are a melting point of 111°C and 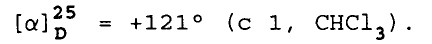
[Senuma et al., Chem. Pharm. Bull. 37 (1989) 3204].

### Example 3

### Resolution of 1b

The enzyme is immobilized on Celite® as described in example 1. 1.33 g (0.0040 mol) of the compound **1b** ((R**₁** = CH**₃**) and 0.92 ml (0.0080 mol) of acetoneoxime acetate dissolved in 40 ml of diethylether are added to the reaction vessel, which contains 1.25 g of the enzyme immobilized on Celite® (0.2 g of lipase PS). The reaction is stopped after two days at 52 % conversion. The enzyme is separated by filtering and the unreacted enantiomer **1b** and the reaction product **2b** (R = CH**₃**) are separated flash-chromatographically using a mixture of CH**₂**Cl**₂**/toluene/AcOEt (1/1/1) as an eluent. As a result 0.59 g (0.0018 mol) of white crystals of **1b**, with 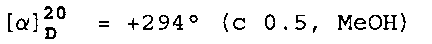
and melting point 107-109°C, are obtained. According to the specific optical rotation the compound is pure (2S,3S)-**1b** (in US-A 4.908.469 the specific optical rotation at of 22 °C is +294° (c 0.5, MeOH) and the melting point is 108-109°C). This reaction also results in 0.68 g (0.0018 mol) of yellowish, amorphous compound (2R,3R)-**2b** (R = CH**₃**),

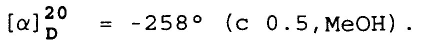

### Example 4

### Resolution of 1c and preparation of (2S,3S)-1b and (2R,3R)-1b

6.0 g (0.016 mol) of the racemic compound **1c** (R**₁** = CH**₃**) and 5.5 g (0.064 mol) of vinyl acetate dissolved in 320 ml of diethylether is added to the reaction vessel, which contains 1.6 g of enzyme (lipase PS). The reaction mixture is shaken or mixed at reaction temperature (44 °C) until the reaction stops at 50 % conversion (48 h). The proceeding of the reaction is followed by HPLC (column: C-18; eluent: MeOH/H**₂**O 70/30). The enzyme is filtered and washed with diethylether, after which the enzyme can be reused. The ether solutions are combined and concentrated. (2S,3S)-**1c** crystallizes from the reaction mixture at -4 °C. The crude product is recrystallized from diisopropylether; 2.61 g (0.0070 mol, 87% of the theoretical yield) of white crystals, 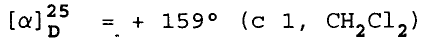
and melting point 128-129°C are obtained. Using flash chromatography the yield of (2S,3S)-**1c** is 50 %, i.e. 100 % of the theoretical yield of the resolution.

From the remaining reaction mixture 3.41 g (0.0080 mol, 100 % of the theoretical yield) of the acetylated reaction product (2R,3R)-**2c** is separated using toluene/AcOEt/CH**₂**Cl**₂** (1/1/1) as an eluent. The product is a yellowish oil, 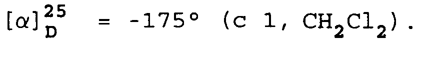
0.5 g (0.0013 mol) of optically pure (+)-(2S,3S)-**1c** is refluxed for 18 h in methanol (30 ml) in the presence of sulfuric acid (0.0052 mol). The reaction mixture is neutralized with sodium hydrogen carbonate and the methanol is evaporated off. The product is recrystallized from diethylether. 0.42 g (0.0013 mol, 98 %) of (2S,3S)-**1b**, 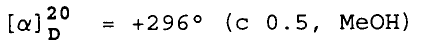
with a melting point of 108-110°C, is obtained as a product. In the US-A 4.908.469 the specific optical rotation of the same ester is stated as +294° (c 0.5, MeOH) at 22 °C. When refluxing correspondingly 1.1 g (0.0026 mol) of (-)-(2R,3R)-**2c** (R = CH**₃**) in methanol (50 ml), 0.811 g (0.0024 mol, 94 %) of the compound (2R,3R)-**1b**, 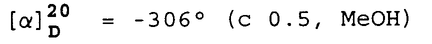
and melting point 108-109°C, is obtained. Based on the specific optical rotation, the resolution products are optically pure.

### Example 5

### Preparation of (2S,3S)-1e and (2R,3R)-2e

### Method A

4.0 g (0.0010 mol) of the racemic compound **1e** (R**₁** = CH**₃**) and 3.5 g (0.0041 mol) of vinyl acetate dissolved in 205 ml of diethylether are added to the reaction vessel containing 1.03 g of enzyme (lipase PS). The reaction mixture is agitated effectively by shaking at room temperature. Liquid chromatography (column: C-18; eluent: MeOH/H**₂**O 80/20) shows that the reaction has been completed (at 50 % conversion) in two days, when according to the HPLC determination (column: Chiralcel OG; eluent: hexane/isopropylalcohol 70/30) the unreacted enantiomer (2S,3S)-**1e** is optically pure (e.e. 100 %). The enzyme is filtered off and washed for reuse. The obtained ether solutions are combined and concentrated and (2S,3S)-**1e** is allowed to crystallize at -4°C. The crude product is recrystallized form diisopropylether. The yield is 1.39 g (70% of the theoretical yield) of white crystals, 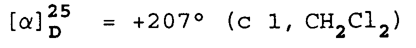
with a melting point of 119-121°C. According to the HPLC determination the product was optically pure.

From the remaining reaction mixture 2.15 g (0.005 mol, 100 %) of an oily product, (2R,3R)-**2e**, is separated flash-chromatographically using toluene/Et**₂**O (2/1) as an eluent. 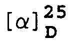
of the product is -193° (c 1, CH**₂**Cl**₂**).

The effect of the reuse of the enzyme filtered off from the reaction mixture in the reaction time of two days is presented in table 2. In each case the reaction proceeds to 50% conversion and (2S,3S)-**1e** is obtained optically pure when the reaction time is extended.

**Table 2**

| Times used | Conversion/% | e.e. (2S,3S)/% |
|---|---|---|
| 1 | 50 | >> 95 |
| 2 | 41 | 94 |
| 3 | 40 | 80 |

### Method B

The enzyme is immobilized on Celite® as described in example 1. 4.64 g (0.0119 mol) of the racemic compound **1e** (R**₁** = CH₃) and 4.1 g (0.047 mol) of vinyl acetate dissolved in 240 ml of diethylether is added to the reaction vessel containing 5.51 g of the above prepared enzyme (lipase PS, 0.475 g) immobilized on Celite®. The reaction stops at 50 % conversion in one day. The work-up of the reaction mixture is similar to method A. The yield of (2S,3S)-**1e** is 1.90 g (82 %) of white crystals, 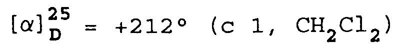
with a melting point of 118-119°C. The yield of (2R,3R)-**2e** (R = CH**₃**) is 2.46 g (93 %),

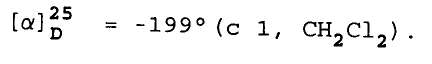

The effect of the reuse of the enzyme preparate filtered off from the reaction mixture in the reaction time of one day is presented in table 3.

**Table 3**

| Times used | Conversion/% | e.e. (2S,3S)/% |
|---|---|---|
| 1 | 48 | 98 |
| 2 | 51 | >> 95 |
| 3 | 50 | >> 95 |

## Claims

1. A method for the resolution of racemic mixtures of *threo*-alkyl-2-hydroxy-3-(4-methoxyphenyl)-3-(2-X-phenylthio)-propionates of formula 1 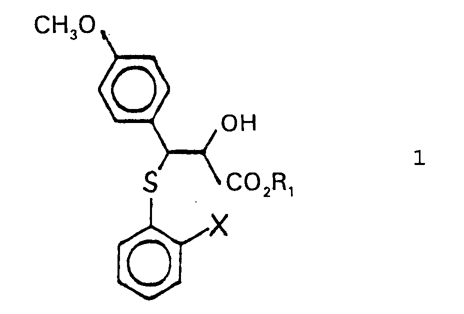 wherein R**₁** is an alkyl group and X is NO**₂**, NH**₂** or an NH-R**₂** group with R**₂** representing an acyl group or an alkoxy carbonyl group, each optionally substituted with one or more halogen atoms,
comprising subjecting said racemic mixtures to reaction with an acylation reagent in an essentially anhydrous organic medium in the presence of a lipase.

2. Method according to claim 1, wherein X is NO**₂**, NH**₂**, NHCHO, NHCOCH**₃**, NHCOCF**₃** or NHCO**₂**CH**₃** and R**₁** is methyl or ethyl.

3. A method according to any one of claims 1 or 2, wherein the lipase is a lipase of *Pseudomonas cepacia*.

4. A method according to anyone of claims 1 or 3, wherein the enzyme is used as a powder or in an immobilized form.

5. A method according to any one of claims 1 to 4, wherein the acylation reagent is selected from acid anhydrides, vinylesters, acetoneoxim acetate or mixtures thereof.

6. A method according to any one of claims 1 to 5, wherein the reaction temperature is in the range of from about 20 to 60°C.

7. A method according to any one of claims 1 to 6, further comprising separating the unreacted (2S,3S)-enantiomer of formula 1 from the acylated (2R,3R)-enantiomer of formula 2 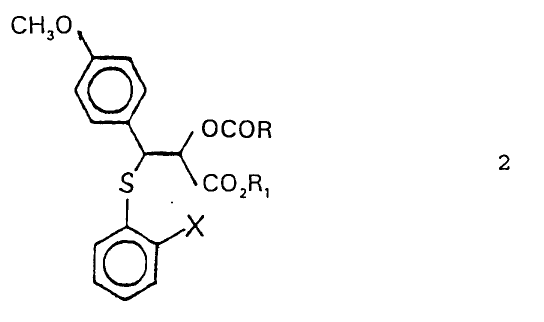 wherein R is an alkyl group and X and R**₁** are as defined in claim 1, by fractional crystallization or chromatography.
